# FASCICULE DE BREVET EUROPEEN

(11) **EP 1 604 045 B1**
(45) Date de publication et mention de la délivrance du brevet: **29.12.2010**
(21) Numéro de dépôt: 04742287.8
(22) Date de dépôt: 18.03.2004
(51) Int. Cl.: C12Q 1/68

(54) **PROCEDE DE PREPARATION DE FRAGMENTS D'ADN ET SES APPLICATIONS**
VERFAHREN ZUR HERSTELLUNG VON DNS-FRAGMENTEN UND DESSEN ANWENDUNGEN
METHOD OF PREPARING DNA FRAGMENTS AND APPLICATIONS THEREOF

(30) Priorité: 18.03.2003 FR 0303294
(43) Date de publication de la demande: 14.12.2005
(73) Titulaire: Commissariat à l'Énergie Atomique et aux Énergies Alternatives, 75015 Paris (FR)
(72) Inventeur: BRACHET, Anne-Gaelle, F-38260 NANTOIN (FR); RIZO, Philippe, F-38700 LA TRONCHE (FR)
(74) Mandataire: Vialle-Presles, Marie José
(86) Numéro de dépôt international: PCT/FR2004/000671
(87) Numéro de publication internationale: WO 2004/085678

(56) Documents cités:
- WO-A-00/75368
- WO-A-02/34939
- WO-A-94/01582
- WO-A-98/10095
- US-A1- 2002 072 055
- US-A1- 2003 008 292
- KATO K: "DESCRIPTION OF THE ENTIRE MRNA POPULATION BY A 3' END CDNA FRAGMENT GENERATED BY CLASS IIS RESTRICTION ENZYMES" NUCLEIC ACIDS RESEARCH, OXFORD UNIVERSITY PRESS, SURREY, GB, vol. 23, no. 18, 1 septembre 1995 (1995-09-01), pages 3685-3690, XP002008304 ISSN: 0305-1048
- SAMBROOK J., RUSSELL D.W.: "Molecular Cloning: A laboratory manual" 2001, COLD SPRING HARBOR PRESS , NEW YORK * page 1.88 - page 1.89 *

## Description

L'invention concerne un procédé de préparation de fragments d'ADN et ses applications, notamment pour l'hybridation d'acides nucléiques.

De nombreuses techniques reposant sur le principe d'hybridation de molécules d'acides nucléiques possédant des séquences complémentaires sont utilisées dans des domaines extrêmement variés de la biologie, notamment pour détecter la présence d'acides nucléiques (ARNm, ADN) à partir d'échantillons à analyser, identifier d'éventuelles variations dans leur séquence ou bien déterminer cette séquence. A titre d'exemple non limitatif, on peut citer : l'analyse des génomes, la cartographie génétique, le génotypage et l'identification d'espèces, de variétés, d'individus (animal, végétal, microorganisme) par recherche d'empreintes génétiques (*DNA finger-printing*), la détection de polymorphisme (SNP ou *Single Nucleotide Polymorphism*), la recherche de mutations ou de gènes liés à des caractères phénotypiques et le miniséquençage, ainsi que l'analyse des transcriptomes, en particulier, l'établissement de profils d'expression géniques.

De manière générale, l'hybridation est effectuée sur des échantillons constitués d'ADN double-brin (extrait d'ADN génomique ou ADNc synthétisé à partir d'un extrait d'ARN). L'ADN double-brin est fragmenté à l'aide d'une ou plusieurs enzymes de restriction, les fragments d'environ 200 à 400 pb sont purifiés, liés de façon covalente - par hybridation (extrémités cohésives) puis ligation à l'aide de ligase (extrémités franches ou cohésives) - à des oligonucléotides double-brin (adaptateurs) dont l'extrémité correspond à la séquence du ou des site(s) de restriction desdites enzymes, puis les fragments sont amplifiés par des réactions d'amplification en chaîne (PCR) à l'aide d'amorces oligonucléotidiques incluant le ou les sites de restriction précédents et dont l'une au moins est marquée à son extrémité 5', de façon à obtenir une quantité suffisante de cibles marquées pour l'hybridation avec la sonde.

Un certain nombre de méthodes mettant en oeuvre un ou plusieurs adaptateurs ont été décrites : Demande EP 0 534 858 au nom de KEYGENE, Demande Internationale PCT WO 02/34939, méthode décrite dans l'article aux noms de Kato K. et al. (N.A.R., 1995, 23, 3685-3690), Demande américaine US 2002/072055, Demande Internationale PCT WO 94/01582 et Demande américaine US 2003/008292.

Les produits PCR ainsi obtenus constituent les cibles qui sont hybridées avec une ou plusieurs sondes immobilisées sur un support approprié (plastique, membrane de nylon, verre, gels, silicium..), chaque sonde étant constituée par une molécule d'acide nucléique simple-brin, dont la séquence est complémentaire de tout ou partie de celle de la cible. Des supports miniaturisés sur lesquels sont fixés de nombreuses sondes (puces à ADN) permettent ainsi de visualiser simultanément des centaines de réactions d'hybridation de fragments cibles (marqués) avec des sondes spécifiques.

D'autres méthodes, ne mettant pas en oeuvre d'étape de PCR, ont également été décrites: Demande Internationale PCT WO 00/75368 et Demande Internationale PCT WO 98/10095.

Aucune de ces méthodes ne permet d'améliorer la sensibilité, la spécificité, la simplicité et la rapidité des méthodes d'hybridation d'acides nucléiques.

De nombreuses applications, en particulier celles qui impliquent la discrimination d'une base entre la séquence de la cible et de la sonde (détection de SNP), requièrent l'utilisation de sondes courtes. Dans ce cas, l'hybridation de produits PCR, c'est-à-dire de cibles de plusieurs centaines de paires de bases avec des sondes de 10 à 20 bases est souvent de mauvaise qualité (signaux faibles, faux négatifs et faux positifs) pour les raisons suivantes :
- la présence de structures secondaires dans la cible diminue l'efficacité de l'hybridation de la sonde, du fait de la diminution de l'accessibilité à la cible et de l'impossibilité d'optimiser les conditions d'hybridation en raison de la présence d'un grand nombre de fragments, de structures différentes, à hybrider avec une même sonde, et
- des réactions d'hybridation non-spécifique ou d'hybridation croisée avec des séquences « non-cibles » possédant des similarités avec la séquence cible conduisent à des faux positifs qui réduisent la capacité à détecter de faibles quantités de séquences spécifiques et la capacité de discrimination de ces techniques, du fait de l'augmentation du bruit de fond.

Ainsi, différentes améliorations ont été proposées pour augmenter la sensibilité (signaux faibles, faux négatifs) et la spécificité (faux positifs) de ces techniques :
- augmentation de la durée d'hybridation (de l'ordre de 12 h à 18 h ; Dai et al., NAR, 2002, 30 (13), e86 ; Ramakrishnan et al., NAR, 2002, 30, 1-12 ; Rodriguez et al., Molecular Biotechnology, 1999, 11, 1 à 12 ; Kane et al., NAR, 2000, 28, 4552-4557) ; cette approche qui permet d'obtenir un bon signal d'hybridation spécifique de la séquence à détecter est incompatible avec les objectifs actuels d'analyse à haut-débit impliquant le traitement rapide d'un grand nombre d'échantillons (miniaturisation, parallélisation des expériences..),
- hybridation de la sonde avec un produit PCR simple brin, obtenu par une réaction de PCR asymétrique (Guo et al., Genome Research, 2002, 12, 447-457) ; cette solution qui permet d'augmenter le signal d'hybridation d'un facteur 4 à 5, implique des étapes supplémentaires de purification du produit PCR double-brin et d'amplification d'un fragment PCR simple-brin marqué.
- optimisation de la longueur et de la composition de la séquence de la sonde, à l'aide de logiciels appropriés ; cette solution qui permet d'améliorer la qualité de l'hybridation en limitant le nombre de structures secondaires et en homogénéisant les températures d'hybridation des sondes ne résout pas les problèmes de réactions croisées liées à la taille et à la structure des cibles.
- utilisation d'oligonucléotides auxiliaires (Rodriguez et al., précité), consistant en une préhybridation de la cible avec de courtes séquences oligonucléotidiques aléatoires et variées avant l'étape d'hybridation avec la sonde, dans le but de rompre les structures secondaires de la cible dans la région à analyser et de limiter la baisse du rendement d'hybridation, due à la présence de séquences redondantes dans la cible ; cette stratégie est coûteuse et n'est pas efficace, dans la mesure où les oligonucléotides ajoutés entrent finalement en compétition avec la sonde et diminuent le signal d'hybridation.

Il ressort de ce qui précède qu'il existe un réel besoin de disposer de méthodes d'hybridation d'acides nucléiques, mieux adaptées aux besoins de la pratique, notamment en ce qu'elles sont rapides, sensibles, spécifiques et simples à mettre en oeuvre. De telles méthodes permettant ainsi d'analyser simultanément un grand nombre d'échantillons sur des supports du type puces à ADN, quelle que soit la technique utilisée, seraient donc parfaitement adaptées à l'ensemble des applications précitées, dans le domaine de la génomique et de la protéomique.

C'est la raison pour laquelle les Inventeurs ont mis au point un procédé de préparation de fragments d'ADN qui permet avantageusement d'obtenir de courts fragments d'ADN et par conséquent d'obtenir une hybridation rapide, efficace et spécifique de molécules d'acides nucléiques (ADN, ARN) ; ledit procédé est utile aussi bien pour la préparation d'ADN-cibles aptes à s'hybrider avec des sondes nucléotidiques et en particulier avec des sondes oligonucléotidiques, que pour la préparation de sondes à ADN, notamment de puces à ADN aptes à s'hybrider avec des acides nucléiques -cibles (ADN, ARN).

La présente invention a ainsi pour objet, un procédé de préparation de fragments d'ADN tel que défini à la revendication 1, caractérisé en ce qu'il comprend au moins les étapes suivantes :
a) la préparation de fragments d'ADN double-brin, à partir d'un échantillon d'acides nucléiques à analyser,
b) la ligation des extrémités desdits fragments d'ADN à un adaptateur oligonucléotidique double-brin (adaptateur AA') comprenant le site de reconnaissance d'une enzyme de restriction dont le site de coupure est situé en aval dudit site de reconnaissance,
c) l'amplification desdits fragments liés audit adaptateur, à l'aide d'un couple d'amorces appropriées, l'une au moins étant éventuellement marquée à son extrémité 5', et
d) la coupure desdits fragments d'ADN à proximité d'une de leurs extrémités, à l'aide de ladite enzyme de restriction, de façon à générer des fragments courts.

Au sens de la présente invention, on entend par fragment court, un fragment inférieur à 100 bases ou 100 paires de bases, de préférence d'environ 20 à 50 bases ou paires de bases.

Le procédé de préparation de fragments d'ADN selon l'invention permet avantageusement d'obtenir des fragments courts c'est-à-dire d'une longueur équivalente à celle des sondes oligonucléotidiques ; l'utilisation de tels fragments courts comme cibles ou sondes dans des techniques d'hybridation, présente les avantages suivants par rapport aux techniques d'hybridation de l'art antérieur :

### • sensibilité et spécificité

La sensibilité et la spécificité de l'hybridation sont augmentées du fait de :
- la diminution des réactions d'hybridation croisée et des faux-positifs, par élimination des « séquences non-cibles »,
- l'augmentation du signal d'hybridation par diminution des structures secondaires de l'ADN,
- l'harmonisation des conditions d'hybridation (température),
- la pureté de l'ADN (élimination de l'enzyme, des tampons et des longs fragments d'ADN restants.)

### • simplicité

La préparation de fragments d'ADN (cible ou sonde) comprend des étapes simples à mettre en oeuvre (digestion enzymatique, ligation et amplification PCR). En outre, l'optimisation de l'ADN (cible ou sonde) permet d'obtenir une hybridation de bonne qualité (pas de faux-positifs, peu de bruit de fond...) et donc de minimiser le nombre de contrôles nécessaires et par conséquent de réduire la complexité de la puce.

### • rapidité

La durée d'hybridation est réduite de façon importante et est inférieure à 1 h (environ 15 à 20 min), au lieu de 12 h à 18 h dans les techniques de l'art antérieur.

### • coût peu élevé

Le procédé de préparation d'ADN selon l'invention est peu coûteux, comparé à l'utilisation d'oligonucléotides auxiliaires.

En outré, la réduction de la complexité de la puce permet de réduire le coût de cette dernière.

En raison de ces différents avantages, le procédé de préparation de fragments d'ADN selon l'invention est particulièrement bien adapté à :
- l'analyse rapide d'un grand nombre d'échantillons d'ADN-cible sur des puces à ADN et ce, quelle que soit la technique d'hybridation utilisée, et par conséquent les applications envisagées (miniséquençage, génotypage, recherche de polymorphisme par SNP, établissement de profils d'expression géniques),
- la préparation de sondes de taille réduite et contrôlée à partir d'ARN ou d'ADN génomique, notamment pour la fabrication de puces à ADN sur lesquelles sont immobilisées lesdites sondes.

Conformément au procédé de l'invention, les étapes a) et b) sont réalisées simultanément.

Conformément au procédé de l'invention, les fragments d'ADN double-brin de l'étape a) sont obtenus par les techniques classiques connues en elles mêmes. Par exemple, l'ADN génomique extrait de l'échantillon à analyser est fragmenté de façon aléatoire à l'aide d'une ou plusieurs endonucléase(s) (enzyme de restriction), sélectionnée(s) en fonction de leur fréquence de coupure de l'ADN à analyser, de façon à obtenir des fragments inférieurs à 1000 pb, de l'ordre de 200 à 400 pb. L'ARN (ARNm, ARN génomique d'un microorganisme...) est extrait de l'échantillon à analyser, converti en ADNc double-brin par rétro-transcription, puis fragmenté de façon analogue à l'ADN génomique. Parmi les endonucléases utilisables pour couper l'ADN de mammifères, on peut citer les enzymes de restriction dont le site de reconnaissance et le site de clivage de l'ADN sont confondus comme les enzymes de restriction de type II, telles que de façon non-limitative : *EcoR I, Dra I, Ssp I, Sac I, BamH I, BbvC I, Hind III, Sph I, Xba I et Apa I.*

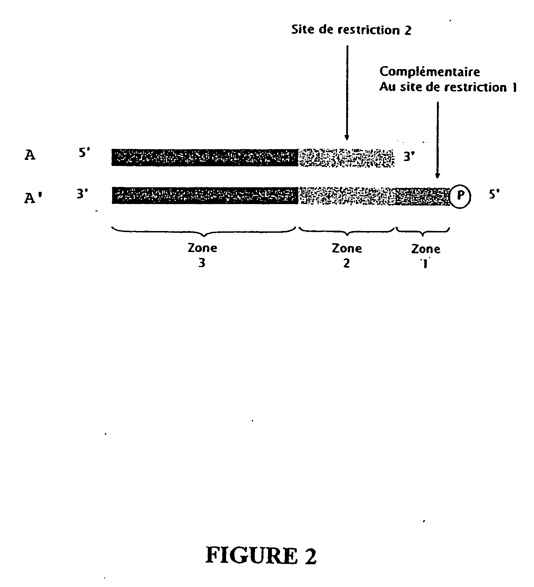

Conformément au procédé de l'invention, l'adaptateur de l'étape b) est un oligonucléotide d'au moins 6 pb, formé de deux brins complémentaires (A et A', figure 2) comprenant le site de reconnaissance d'une enzyme de restriction (zone 2) dont le site de coupure est situé en aval du site de reconnaissance. Parmi ces enzymes de restriction, on peut citer les enzymes de restriction de type IIS ou F telles que de façon non-limitative : *Bpm I, Bsg I* et *BpuE I* qui coupent 16 nucléotides en aval de leur site de reconnaissance, et *Eci I, BsmF I, Fok I, Mme I et Mbo II* qui coupent respectivement 11, 10, 9, 20 et 8 nucléotides en aval de leur site de reconnaissance. De préférence, ledit adaptateur est formé de l'association de deux oligonucléotides complémentaires dont la séquence est respectivement celle des brins A et A' tels que définis ci-dessus. Ledit adaptateur est lié aux extrémités dudit fragment d'ADN par tout moyen approprié, connu en lui-même, notamment à l'aide d'une ligase à ADN, telle que la T4 ligase.

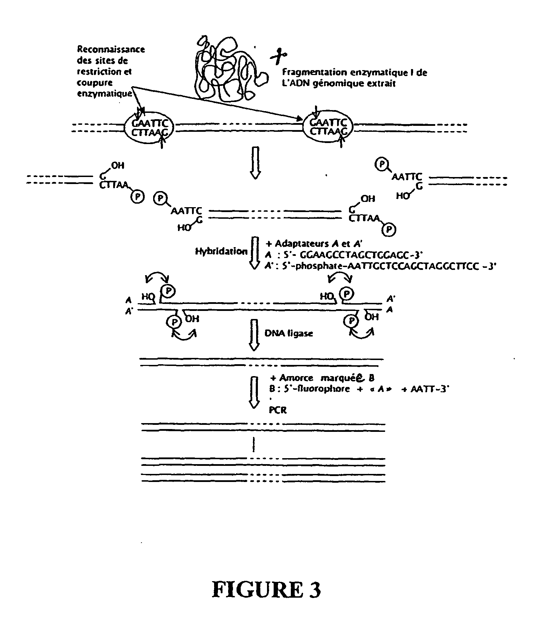

Conformément au procédé de l'invention, l'amplification de l'étape c) est réalisée à l'aide d'une amorce comprenant la séquence de l'oligonucléotide A de l'adaptateur. Par exemple, la séquence de l'amorce est, soit celle de l'oligonucléotide A, soit celle de ce dernier, additionnée en 3' des bases correspondants à la séquence protrusive des extrémités du fragment de l'étape a), générée par l'endonucléase utilisée à l'étape a), telle que définie ci-dessus (amorce B, figure 3). Conformément au procédé de l'invention, la coupure à l'extrémité du fragment d'ADN double-brin de l'étape d), permet d'obtenir de courts fragments d'ADN susceptibles de contenir la séquence à détecter (séquence informative) par hybridation avec une sonde nucléoti-dique spécifique, en particulier un oligonucléotide complémentaire de ladite séquence informative.

La Demande décrit un procédé qui comprend une étape additionnelle de purification des fragments inférieurs à 1000 pb, préalablement à l'étape b) de ligation. Ladite purification est effectuée par tout moyen approprié connu en lui-même, notamment par séparation des produits de digestion obtenus en a) par électrophorèse en gel d'agarose, visualisation des bandes correspondant aux différents fragments obtenus, prélèvement de(s) bande(s) du gel correspondant aux fragments inférieurs à 1000 pb et extraction desdits fragments d'ADN double-brin selon les techniques classiques.

Selon un autre mode de mise en oeuvre avantageux du procédé selon l'invention, ledit adaptateur d'au moins 6 pb (étape b) comprend, en amont du site de reconnaissance (zone 2), une zone 3 d'au moins 6 paires de bases ; une telle zone permet d'améliorer l'hybridation par allongement de l'adaptateur (figure 2). La séquence de la zone 3 est sélectionnée par tout moyen approprié connu en lui-même, notamment à l'aide de logiciels de prédiction de séquences appropriés permettant d'optimiser la longueur, la structure et la composition des oligonucléotides (pourcentage de GC, absence de structures secondaires et/ou d'auto-appariement...).

Selon encore un autre mode de mise en oeuvre avantageux du procédé selon l'invention, ledit adaptateur (étape b) comprend sur l'un des brins (A ou A'), en aval du site de reconnaissance (zone 2), une zone 1 complémentaire de la séquence protrusive des extrémités du fragment de l'étape a), générée par l'endonucléase utilisée à l'étape a), telle que définie ci-dessus (figure 2). De préférence, ledit adaptateur comprend au moins une base située entre la zone 1 et la zone 2, différente de celle qui dans ledit site de restriction, est immédiatement adjacente à la séquence complémentaire précédente ; cette base permet de ne pas reconstituer ledit site de restriction après la ligation de l'adaptateur à l'étape b) et donc d'éviter le clivage de l'adaptateur lié à l'extrémité dudit fragment d'ADN double-brin.

Selon encore un autre mode de mise en oeuvre avantageux du procédé selon l'invention, ledit adaptateur (étape b) comprend un résidu de phosphate lié de façon covalente à l'extrémité 5' du brin A' ; ce résidu de phosphate permet à une enzyme (par exemple une ligase à ADN telle que la T4 ligase) de lier ledit adaptateur aux extrémités 3'-OH du fragment d'ADN double-brin, par l'intermédiaire d'une liaison phosphodiester.

Selon encore un autre mode de mise en oeuvre avantageux du procédé selon l'invention, l'une des amorces (étape c) est liée à son extrémité 5' à un marqueur approprié permettant la détection d'hybrides d'acide nucléique (ADN-ADN, ADN-ARN), par exemple un fluorophore.

Selon encore un autre mode de mise en oeuvre avantageux du procédé selon l'invention, lesdites amorces (étape c) contiennent à leur extrémité 3', plusieurs bases spécifiques de séquence(s) informative(s) à détecter, de façon à amplifier seulement certains des fragments (amplification différentielle), notamment pour éviter la saturation de la puce par un nombre trop important de fragments d'ADN-cible.

Selon encore un autre mode de mise en oeuvre avantageux du procédé selon l'invention, l'un des brins du produit amplifié à l'étape c) est protégé à son extrémité 5' par un marqueur approprié ; il est ainsi possible d'éliminer le brin complémentaire par l'action d'une phosphatase puis d'une 5' exonucléase. Le brin marqué n'est pas détruit par l'enzyme, le marqueur empêchant la progression de l'exonucléase le long du brin et donc sa digestion.

Selon encore un autre mode de mise en oeuvre avantageux du procédé selon l'invention, il comprend une étape additionnelle e) d'obtention, par tout moyen approprié, de fragments simple-brin à partir des fragments courts obtenus à l'étape d).

Selon encore un autre mode de mise en oeuvre avantageux du procédé selon l'invention, il comprend une étape additionnelle e') de purification, par tout moyen approprié, des fragments courts obtenus à l'étape d) ou une étape f) de purification des fragments simple-brin obtenus à l'étape e).

Conformément au procédé de l'invention, l'obtention de fragments d'ADN simple-brin est réalisée par tout moyen approprié connu en lui-même, par exemple par action d'une phosphatase alcaline puis d'une 5' exonucléase.

Conformément au procédé de l'invention, la purification des fragments courts, éventuellement simple-brin, est réalisée par tout moyen approprié connu en lui-même, par exemple : la chromatographie d'exclusion, la filtration, la précipitation à l'aide de mélanges d'éthanol et d'acétate d'ammonium ou de sodium.

La demande décrit un fragment court d'ADN simple-brin, susceptible d'être obtenu par le procédé tel que défini ci-dessus, caractérisée en ce qu'il présente une longueur inférieure à 100 bases ou paires de bases et en ce qu'il comprend au moins une séquence informative bordée à ses extrémités 5' et 3', respectivement par le site de reconnaissance et le site de clivage d'une enzyme de restriction coupant à distance de son site de reconnaissance.

La séquence informative ou séquence cible correspond à la séquence d'un échantillon d'acides nucléiques à analyser, qui est détectée spécifiquement par la sonde utilisée pour l'hybridation ; ladite séquence informative représente par exemple un marqueur génétique utile pour la détection d'une espèce, d'une variété ou d'un individu (animal, végétal, microorganisme) ou d'une zone de polymorphisme ou bien un marqueur d'ADNc spécifique d'une protéine, utile pour l'étude de transcriptomes et l'établissement de profils d'expression géniques.

Ledit fragment court d'ADN simple-brin peut comprendre également, en amont et/ou en aval du site de reconnaissance de ladite enzyme de restriction, les séquences correspondant à la zone 1 et à la zone 3, telles que définies ci-dessus.

Ledit fragment court d'ADN simple-brin peut être marqué à son extrémité 5' par un marqueur approprié permettant la détection d'hybrides ADN-ADN, par exemple un fluorophore.

Ledit fragment court d'ADN simple-brin peut être immobilisé sur un support approprié. Les supports sur lesquels on peut immobiliser des acides nucléiques sont connus en eux-mêmes ; à titre d'exemple non-limitatif on peut citer ceux qui sont réalisés dans les matériaux suivants : plastique, nylon, verre, gel (agarose, acrylamide...) et silicium.

De préférence, ledit fragment d'ADN est immobilisé sur un support miniaturisé, du type puce à ADN.

La demande décrit une puce à ADN, caractérisée en ce qu'elle comprend un fragment court d'ADN simple-brin tel que défini ci-dessus.

La demande décrit une méthode d'hybridation d'acides nucléiques, caractérisée en ce qu'elle met en oeuvre :
- une sonde ou une cible constituées par un fragment court d'ADN simple-brin tel que défini ci-dessus, et/ou
- une sonde constituée par un fragment court d'ADN double-brin formé de l'association du fragment court d'ADN simple-brin tel que défini ci-dessus et de sa séquence complémentaire.

Un kit pour la mise en oeuvre d'une méthode d'hybridation est aussi décrit, caractérisé en ce qu'il comprend au moins un fragment d'ADN (cible ou sonde), tel que défini ci-dessus et une molécule d'acide nucléique complémentaire dudit fragment d'ADN, notamment une sonde oligonucléotidique.

La demande décrit l'utilisation d'un adaptateur tel que défini ci-dessus, pour la préparation de fragments courts d'ADN simple-brin tels que définis ci-dessus.

La présente invention a également pour objet l'utilisation d'une amorce telle que définie dans la revendication 10, pour la préparation de fragments courts d'ADN simple-brin tels que définis ci-dessus.

La présente invention a également pour objet un adaptateur formé d'un oligonucléotide double-brin (AA') d'au moins 10 pb comprenant de 5' en 3' (figure 2) :
- une zone 3 d'au moins 6 pb, telle que définie ci-dessus,
- une zone 2 comprenant le site de reconnaissance d'une enzyme de restriction dont le site de coupure est situé en aval du site de reconnaissance,
- une zone 1 complémentaire de la séquence protrusive des extrémités du fragment de l'étape a) du procédé tel que défini ci-dessus, générée par l'endonucléase utilisée à l'étape a) telle que définie ci-dessus,
- au moins une base située entre la zone 1 et la zone 2, différente de celle qui dans ledit site de restriction de ladite endonucléase utilisée à l'étape a), est immédiatement adjacente à la séquence complémentaire de ladite zone 1, et
- un résidu de phosphate lié de façon covalente à l'extrémité 5' du brin A.'

La présente invention a également pour objet une amorce, telle que défini à laser 10, caractérisée en ce qu'elle comprend la séquence de l'oligonucléotide A de l'adaptateur. De préférence, la séquence de ladite amorce est sélectionnée dans le groupe constitué par : la séquence de l'oligonucléotide A et la séquence de ce dernier, additionnée en 3' des bases correspondants à la séquence protrusive des extrémités du fragment de l'étape a), générée par l'endonucléase utilisée à l'étape a), telle que définie ci-dessus (amorce B, figure 3).

La présente invention a également pour objet un kit tel que défini a la revendication 13 pour la mise en oeuvre du procédé tel que défini ci-dessus.

La description qui va suivre se réfère à des exemples de mise en oeuvre du procédé de préparation de fragments d'ADN selon l'invention et de son utilisation pour l'hybridation d'acides nucléiques, en particulier à des sondes oligonucléotidiques, ainsi qu'aux dessins annexés dans lesquels :

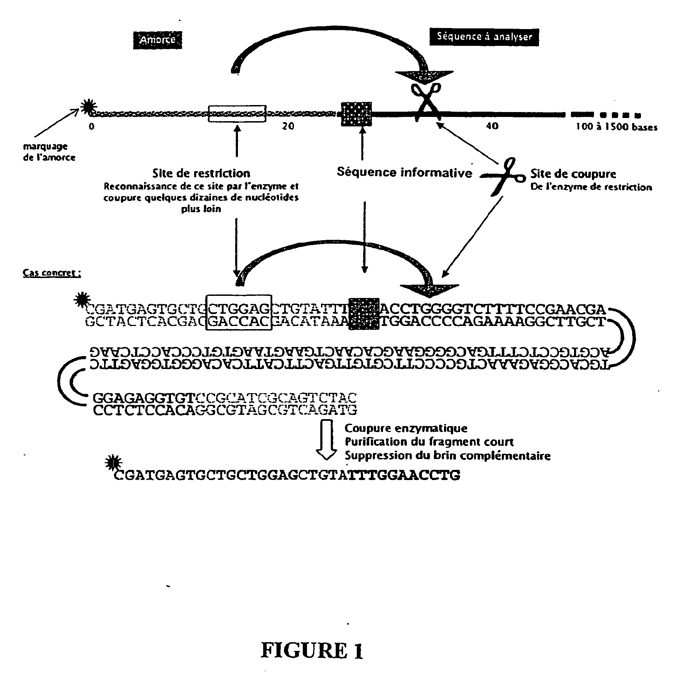

- la figure 1 illustre le principe du procédé de préparation de fragments d'ADN (cible ou sonde) selon l'invention.
- la figure 2 illustre la structure générale de l'adaptateur (AA')
- la figure 3 illustre un exemple des étape a) à c) du procédé de préparation de fragments d'ADN selon l'invention :
- étape a): les fragments d'ADN double-brin sont générés par coupure avec *EcoR I* qui reconnaît le site GAATTC,
- étape b) : l'adaptateur AA' (16/20 pb), comprend respectivement de 5' en 3': une séquence de 10 paires de bases (zone 3 : séquence 5' GGAAGCCTAG 3'sur le brin A), le site de reconnaissance de l'enzyme *Bpm I* (zone 2 : séquence 5' CTGGAG 3' sur le brin A), ainsi que la séquence complémentaire du site *EcoR I* et une paire de bases supplémentaire, ainsi qu'un résidu de phosphate à l'extrémité 5' du brin A' (zone 1 : séquence 5'phosphate-AATTG sur le brin A'). L'ADN ligase permet la liaison de l'adaptateur aux extrémités cohésives des fragments *EcoR I* par l'intermédiaire de liaisons phosphodiester, et
- étape c) : les fragments liés à l'adaptateur sont amplifiés par PCR à l'aide de l'amorce B (21 bases), l'amorce B étant marquée à son extrémité 5' par un fluorophore.

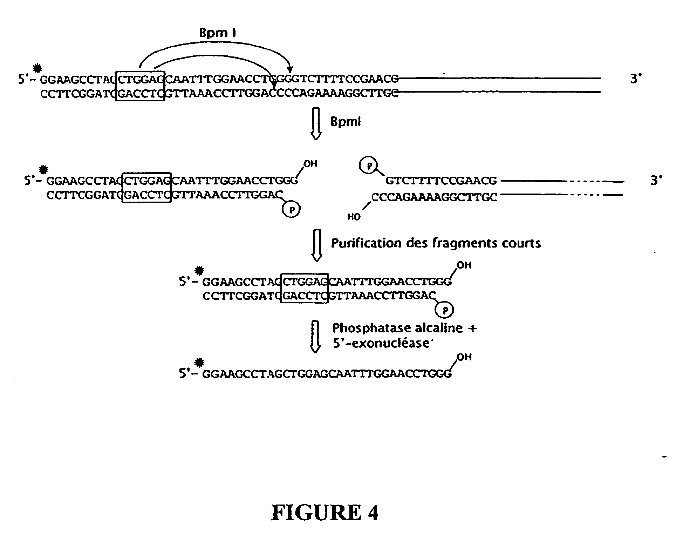

- la figure 4 illustre un exemple des étapes d) et e) du procédé de préparation d'ADN- cibles selon l'invention : les fragments marqués obtenus à l'étape c) sont coupés à leur extrémité 5', à l'aide de l'enzyme *Bpm I* qui coupe 16 nucléotides en aval du site de reconnaissance (14 nucléotides en aval sur le brin complémentaire), de façon à générer des fragments courts (32/30 pb) qui sont purifiés, puis le brin complémentaire non-marqué est éliminé par digestion successivement, par une phosphatase alcaline et une 5' exonucléase. Les fragments d'ADN marqués ainsi obtenus présentent une longueur de 32 bases comprenant 12 bases de séquence informative, spécifique des acides nucléiques à analyser.

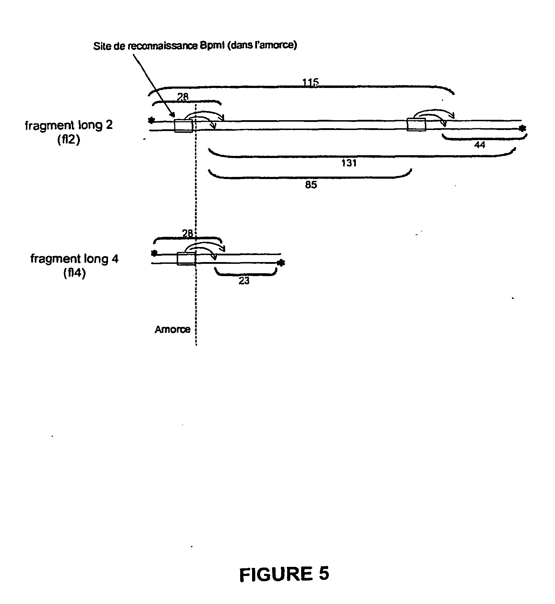

- la figure 5 représente la carte de restriction des fragments longs fl2 et fl4 par *Bpm I,*

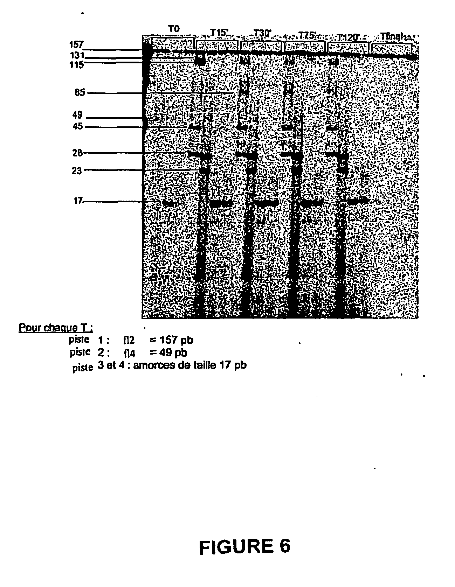

- la figure 6 représente le profil en gel de polyacrylamide (20 %), des fragments courts radiomarqués obtenus après coupure des fragments longs fl2 et fl4 par *Bpm I.* Pour chaque temps (T) d'incubation avec l'enzyme de restriction *Bpm I* (0, 15, 30, 75, 120 minutes et T final), la piste 1 correspond à fl2 (157pb) la piste 2 à fl4 (49pb) et les pistes 3 et 4 aux amorces (17pb).

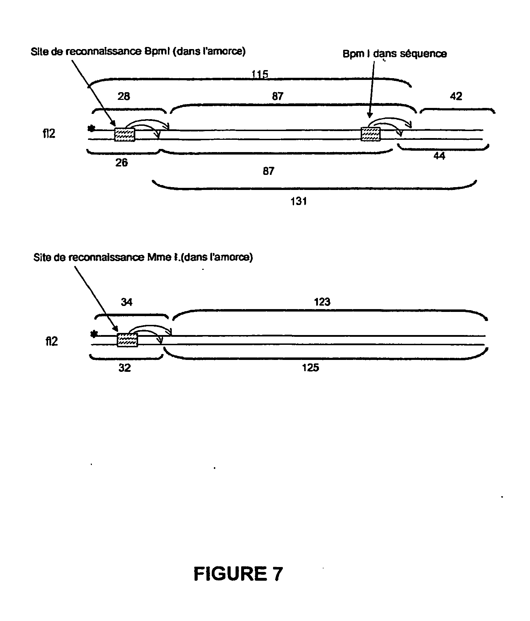

- la figure 7 représente la carte de restriction du fragment long fl2 par *Bpm I* et *Mme I.*

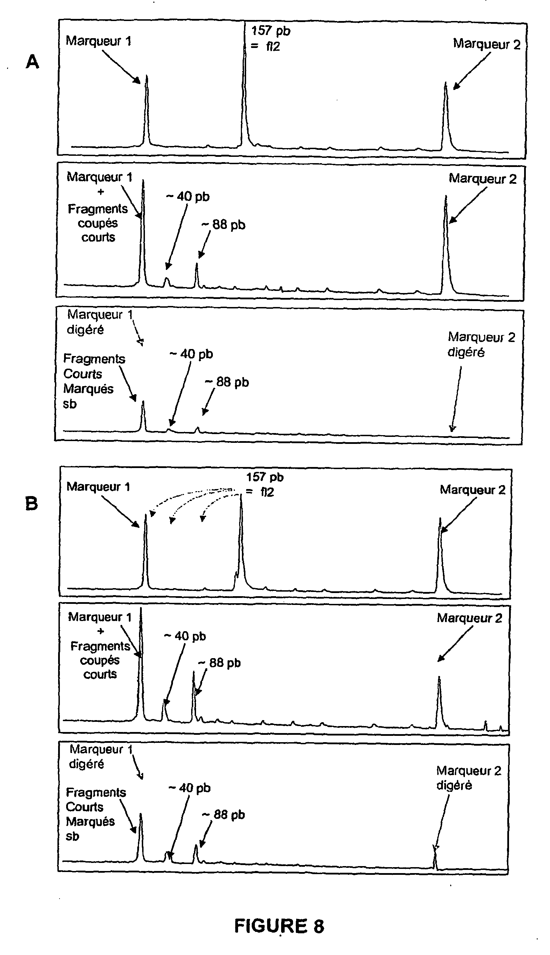

- les figures 8A et 8B représentent le profil des fragments obtenus après coupure par *Bpm I,* du fragment long fl2 marqué en 5' par Cy3 (panneau central en A) ou fam *(fluorescein acetoxymethyl-ester)* (panneau central en B). Le panneau supérieur en A et B correspond au profil du fragment fl2 non-coupé par *Bpm I.* Le panneau inférieur en A et B correspond au profil du fragment fl2 coupé par *Bpm I* et digéré par la phosphatase alcaline et l'exonucléase 5' PDE II.

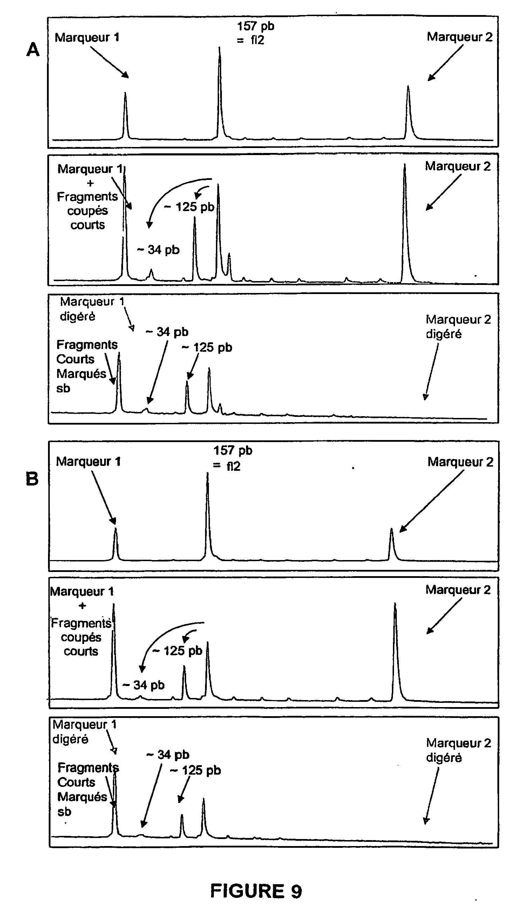

- les figures 9A et 9B représentent le profil des fragments obtenus après coupure par *Mme I,* du fragment long fl2 marqué en 5' par Cy3 (panneau central en A) ou fam (panneau central en B). Le panneau supérieur en A et B correspond au profil du fragment fl2 non-coupé par *Mme I.* Le panneau inférieur en A et B correspond au profil du fragment fl2 coupé par *Mme I* et digéré par la phosphatase alcaline et l'exonucléase 5' PDE II.

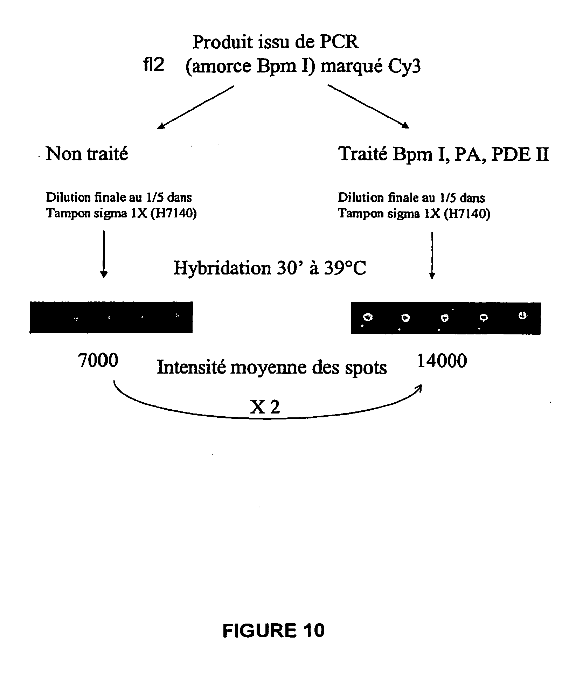

- la figure 10 illustre l'analyse de l'intensité du signal d'hybridation d'une cible courte double-brin ou simple-brin, par comparaison avec une cible longue double-brin.

### Exemple 1 : Préparation de fragments d'ADN (cible ou sonde) selon le procédé de l'invention.

La préparation des acides nucléiques, les digestions enzymatiques, les ligations, les amplification PCR et la purification des fragments ainsi obtenus, ont été réalisées en utilisant les techniques classiques, selon les protocoles standards tels que ceux décrits dans Current Protocols in Molecular Biology (Frederick M. AUSUBEL, 2000, Wiley and son Inc, Library of Congress, USA*).*

De manière plus précise, des fragments d'ADN ont été préparés de la façon suivante :

L'ADN génomique a été extrait à partir de sang de bovin *(Bos taurus),* à l'aide du kit *PAXgene Blood DNA* (référence 761133, QIAGEN), selon les instructions du fabricant.

Les adaptateurs et les amorces suivants ont été synthétisés par MWG BIOTECH :
- **adaptateur**
   - brin A : 5'-GGAAGCCTAGCTGGAGC-3' (SEQ ID NO : 1)
   - brin A' : 5' -P-AATTGCTCCAGCTAGGCTTCC-3'(SEQ ID NO : 2)
- **amorce**
   B : 5'-Cy- GGAAGCCTAGCTGGAGCAATT-3'(SEQ ID NO : 3)
L'ADN génomique purifié (5 µg) et l'adaptateur (5 µg) ont été incubés, 3 h à 37 °C, dans 40 µl de tampon 50 mM Tris-HCl, pH 7,5, 10 mM MgCl₂, 50 mM NaCl, 10 mM DTT, 1 mM ATP et 1 mg BSA, contenant 50 UI *d'EcoR I* et 2 UI de T4 ADN-ligase. Les fragments d'ADN liés à leurs extrémités à l'adaptateur AA' ainsi obtenus, ont été amplifiés par PCR à l'aide de l'amorce B dans un volume réactionnel de 50 µl contenant : 1 ng de fragments d'ADN, 150 ng de l'amorce et 2 UI d'AmpliTaq GOLD® (PERKIN ELMER) dans un tampon 15 mM Tris HCl, pH 8,0, 10 mM KCl, 5 mM MgCl₂ et 200 µM dNTPs. L'amplification a été réalisée dans un thermocycleur, pendant 35 cycles comprenant : une étape de dénaturation à 94°C pendant 30 s, suivie d'une étape d'hybridation à 60°C pendant 30 s et d'une étape d'extension à 72°C pendant 2 min. Les fragments amplifiés par PCR ont été purifiés à l'aide du kit *MinElute PCR Purification* (référence LSKG ELO 50, QIAGEN), en suivant les instructions du fabricant.

Les fragments amplifiés ont été digérés 1 h à 37°C, dans un mélange réactionnel de 40 µl contenant 2,5 UI de *Bpm I* (NEB) dans un tampon 50 mM Tris-HCl, pH 7,9, 100 mM NaCl, 10 mM MgCl₂, 1 mM DTT et 100 µg/ml BSA.

Les enzymes et les tampons ont ensuite été éliminés par filtration (Microcon YM3, MILLIPORE) et l'ADN retenu sur le filtre a été élué à l'aide du kit Micropure-EZ (MILLIPORE), puis les fragments courts ont été purifiés par filtration (Microcon YM 30, MILLIPORE) ; les fragments d'ADN de moins de 100 pb correspondant à l'éluat, les fragments de plus grande taille étant retenus sur le filtre.

Les fragments courts ont ensuite été digérés, 1 h à 37°C, dans un volume réactionnel de 40 µl contenant 5 UI de phosphatase alcaline et 3UI de 5'-exonucléase dans un tampon 500 mM Tris-HCl-1 mM EDTA, pH 8,5 puis la réaction a été stoppée par chauffage à 90°C pendant 3 min.

Les fragments d'ADN-cible simple-brin marqués par un fluorophore, ainsi obtenus ont été conservés en vue d'une utilisation ultérieure pour l'hybridation avec des sondes nucléotidique(s).

### Exemple 2 : Analyse des fragments courts obtenus par digestion avec une enzyme de restriction de type IIS selon le procédé de l'invention.

### 1) Préparation de fragments longs marqués contenant à l'une de leur extrémité, le site de reconnaissance d'une enzyme de restriction de type IIS.

### a) Amplification par PCR

Des fragments longs dénommés fl1, fl2, fl4 et fl5, présentant respectivement les séquences SEQ ID NO : 4 à SEQ ID NO : 7 ont été amplifiés par réaction de polymérisation en chaîne (PCR), à l'aide des couples d'amorces suivants :
- fl1
   - amorce sens : 5' CGATGAGTGCTGACCGA 3' (SEQ ID NO : 8)
   - amorce anti-sens : 5' GTAGACTGCGATGCG 3' (SEQ ID NO : 9)
- fl2. fl4 et fl5
   - amorce sens: 5' CGATGAGTGCTGA 3' (SEQ ID NO : 10)
   - amorce anti-sens: 5' GTAGACTGCGATGCG 3' (SEQ ID NO : 9)

Le site de reconnaissance de l'enzyme de restriction *Bpm I* (5'CTGGAG3') ou *Mme I* (5'TCCPuAC3') a été introduit à l'extrémité 5' des produits ainsi obtenu, par une seconde amplification PCR à l'aide du couple d'amorces suivant :
- *Bpm I*
   - amorce sens : 5' CGATGACTGGAGACCGA 3' (SEQ ID NO : 11)
   - amorce anti-sens : 5' GTAGACTGCGATGCG 3' (SEQ ID NO : 9)
- *Mme I*
   - amorce sens : 5' CGATGAGTTCCGACCGA 3' (SEQ ID NO : 12)
   - amorce anti-sens : 5' GTAGACTGCGATGCG 3' (SEQ ID NO : 9)

### b) Marquage

Les fragments longs modifiés obtenus en a) ont été marqués à leur extrémité 5', soit par du γ³²P-ATP, soit par un fluorophore tel que la cyanine 3 (Cy3) ou le fam.

De manière plus précise, les produits PCR (2 µl) obtenus en a) sont dénaturés par chauffage à 80°C, transférés immédiatement dans de l'azote liquide, puis 1 µl d'un mélange de marquage contenant de la polynucléotide kinase (PNK, 30UI) et 2 µL d'ATPγ³²P dans un volume final de 50 µl de tampon de cette enzyme sont ajoutés et le marquage est réalisé pendant 30 minutes à 37°C. Les produits radiomarqués sont ensuite purifiés sur une colonne d'exclusion G25.

### 2) Analyse des fragments courts radiomarqués obtenus après coupure par Bpm I

### a) Coupure par Bpm I

Les produits PCR radiomarqués purifiés comme ci-dessus, sont dissous dans du tampon de l'enzyme *Bpm I* (5X, 4 µL), puis hybridés à nouveau par chauffage à 80°C puis retour lent à la température ambiante ; 16 µL d'H₂O sont ensuite ajoutés et 4 µL du mélange final (20 µl) sont prélevés pour la digestion. L'enzyme de restriction est alors ajoutée (2 unités soit 1 µL ; New England Biolabs) ainsi que 0,2 µL de sérum albumine bovine (10 mg/mL) et 1 µL de tampon de l'enzyme, dans un volume final de 10 µL. Des fractions aliquotes de 2 µL sont prélevées à différents temps (15, 30, 75 et 120 minutes) pour suivre l'avancement de la réaction. Les réactions sont stoppées par ajout de 2 µL d'une solution de formamide contenant du bleu de bromophénol et du xylène cyanol puis chauffage du mélange pendant 3 minutes à 80°C. Les 2 µL de produit de clivage restant sont digérés comme précisé ci-dessous.

### b) Digestion par la phosphatase alcaline (PA) et l'exonucléase 5' (PDE II)

Le produit de clivage par *Bpm I* restant (2 µL) est ensuite traité avec de la phosphatase alcaline (P5521 Sigma, 1000U/40 µL dans du tampon sulfate d'ammonium 3,2 M, pH7) pendant 15 minutes à 37°C, puis avec du PDE II (P9041 Sigma, 10⁻¹ U/µL dans du tampon citrate d'ammonium 2M, pH 5,5) pendant de 30 minutes à 37°C. Le produit de digestion ainsi obtenu correspond ci-après au temps T final.

### c) Analyse en gel de polyacrylamide des produits de clivage obtenus

La figure 5 représente la carte de restriction des fragments longs fl2 et fl4 par *Bpm I*. De manière plus précise, la coupure de fl2 par *Bpm I* génère les fragments suivants, à savoir : des fragments de 28 et 131 paires de bases (pb) par coupure en aval du site de reconnaissance de *Bpm I* situé en 5', généré par PCR, des fragments de 115 et 44 pb par coupure en aval du deuxième site de *Bpm I* (site interne présent uniquement dans fl2), et des fragments de 28, 85 et 44 pb par coupure en aval des deux site de reconnaissance précédents. La coupure de fl4 par *Bpm I* génère des fragments de 28 et 23 nucléotides.

L'analyse en gel d'acrylamide (20 %) de la cinétique de coupure des fragments fl2 et fl4 par *Bpm I* (figure 6), montre la présence de fragments de 131, 115, 85, et 45 pb environ pour fl2 et de fragments de 23 et 28 pb pour fl4, indiquant que la coupure par l'enzyme *Bpm I* est efficace dès 15 minutes. La disparition du signal au temps T final indique que la digestion par la phosphatase alcaline et l'exonucléase 5' PDE II est efficace.

### 3) Analyse des fragments courts marqués par un fluorophore obtenus après coupure par Bpm I ou Mme I

Les fragments courts marqués par un flurophore, obtenus après coupure par *Bpm I* ou *Mme I* sont analysés à l'aide d'un bioanalyseur (Agilent) qui comprend la séparation de l'ADN par électrophorèse sur gel et la détection des différents fragments par mesure de la quantité de fluorescence émise par un agent intercalant spécifique de l'ADN double-brin ; cette technique ne permet pas de détecter les fragments d'ADN double-brin la taille est inférieure à 25 pb et les fragments d'ADN simple-brin.

### a) protocole

Le fragment fl2 modifié, marqué par un fluorophore (4 µL), préparé comme ci-dessus est incubé pendant 3 heures à 37°C dans un mélange réactionnel de 10 µL contenant 1 µL de tampon numéro 3 (10X ; New England Biolabs), 4,4 µL d'H₂O, 0,2 µL de sérum albumine bovine et 0,5 µL de *Bpm I* (1 unité ; New England Biolabs). Cinq microlitres du produit de clivage sont analysés sur le bioanalyseur et les 5 µL restants sont traités avec 2 µL de phosphatase alcaline (P5521 Sigma, 1000U/40 µL dans du tampon sulfate d'ammonium, 3,2M, pH7) pendant 15 minutes à 37°C, suivi d'une incubation de 30 minutes à 37°C avec 0,5 µL de PDE II (P9041 Sigma, 10⁻¹ U/ µL dans du tampon citrate d'ammonium 2M, pH 5,5).

Alternativement, le fragment fl2 modifié, marqué par un fluorophore (4 µL), préparé comme ci-dessus est incubé pendant 3 heures à 37°C dans un mélange réactionnel de 10 µL contenant 1 µL de tampon numéro 4 (10X; New England Biolabs), 3,5 µL d'H₂O, 1 µL de SAM (S-adénosylméthionine) et 0,5 µL de *Mme I*(1 unité). Cinq microlitres de ce produit de digestion sont analysés sur le bioanalyseur et les 5 µL restants sont traités de la phosphatase alcaline et de la PDE II comme ci-dessus.

En outre, des marqueurs de poids moléculaire sont ajoutés au mélange avant l'analyse à l'aide du bioanalyseur, de façon à identifier la taille des fragments générés après coupure par les enzymes de restriction.

### b) Résultats

La carte de restriction du fragment fl2 par *Bpm I* et *Mme I* est présentée à la figure 7.

Les figures 8 et 9 illustrent le profil des fragments obtenus après coupure, respectivement par *Bpm I* et *Mme I,* du fragment long fl2 marqué en 5' par Cy3 (en A) ou fam (en B).

Par comparaison avec le profil contrôle (en l'absence d'enzyme de restriction ; panneau supérieur), les résultats montrent que la coupure par *Bpm I* est totale (figures 8A et 8B ; panneau central), alors que la coupure par *Mme I* est partielle (figures 9A et 9B ; panneau central). En outre, le profil des fragments marqué en 5' par Cy3 (en A) ou fam (en B), coupés par *Bpm I* ou *Mme I* et digérés par la phosphatase alcaline et l'exonucléase 5' montre une diminution du signal (figures 8A, 8B, 9A et 9B ; panneau inférieur), indiquant une digestion de l'ADN par ces enzymes, mais seulement partielle. Toutefois, ce type d'analyse qui est spécifique de l'ADN double-brin ne permet pas de vérifier la digestion totale du brin d'ADN non-couplé en 5' au fluorophore, la protection du brin d'ADN couplé en 5' au fluorophore et la présence du fragment court simple-brin marqué en 5' par un fluorophore qui en résulte.

### Exemple 3 : Utilisation des ADN-cibles pour hybrider des sondes oligonucléotidiques

### 1) Matériels et méthodes

Un support en verre du type puce à ADN (lames Codelink), sur lequel sont immobilisées des sondes oligonucléotidiques dont certaines sont complémentaires aux fragments d'ADN-cible obtenus à l'exemple 1 ou 2, a été préparé selon les techniques connues en elles-mêmes. Lesdits ADN-cibles (1,5 µl) ont ensuite été dilués dans du tampon d'hybridation (H7140, SIGMA; 1,5 µl) et 10 µl ont été déposés sur le support en verre, entre lame et lamelle (lamelle ronde de 12 mm de diamètre). L'hybridation a ensuite été réalisée, en chambre humide dans un thermo-cycle, dans les conditions suivantes : 80°C pendant 3 min, puis la température est abaissée à 50°C par palier de 0,1°C/s et enfin la température est maintenue à 50°C pendant 10 minutes. La réaction d'hybridation est ensuite stoppée par dépôt des lames de verre sur la glace. Alternativement, lL'hybridation est réalisée dans une étuve ventilée à 39°C pendant 30 minute

L'excès de fragments d'ADN-cible non-complémentaires des sondes est ensuite éliminé par des lavages successifs : 1 lavage 20 s à 30 s avec du SSC 2X (SIGMA, S6639), 1 lavage 20 s à 30 s avec du SSC 2X additionné de 0,1 % SDS (L4522, SIGMA), et 3 lavages 20 s à 30 s avec du SSC 0,2X, à + 4°C.

Les lames de verre ont ensuite été séchées et l'hybridation a été visualisée et analysée à l'aide d'un scanner (modèle Genetac, GENOMIC SOLUTION).

### 2) Résultats

L'hybridation des cibles suivantes préparées comme décrit à l'exemple 2 a été comparée :
- fragment fl2 court marqué en 5' par Cy3, généré par coupure par *Bpm I.*
- fragment fl2 court marqué en 5' par Cy3, généré par coupure par *Bpm I*, digéré par la phosphatase alcaline et l'exonucléase 5' PDEII,
- fragment fl2 marqué en 5' par Cy3, non coupé, non digéré (contrôle)

Les résultats de l'analyse comparative (figure 10 et le Tableau I) montrent que l'intensité du signal d'hybridation de la cible longue double-brin est inférieure d'un facteur 2, à celle des fragments courts double-brin. L'intensité d'hybridation est encore augmentée lorsque les fragments courts double-brin sont convertis en simple-brin.

**Tableau I : Analyse comparative de l'intensité du signal d'hybridation des différentes cibles**

| **Intensité** | **Témoin non traité** | ***Bpm I*** | ***Bpm I* + PA + PDE II** |
|---|---|---|---|
| **I max (x 103)** | 13 | 20 | 23 |
| **I moyenne** | 7300 | 13500 | 14300 |

## Revendications

1. Procédé de préparation de fragments d'ADN, **caractérisé en ce qu'**il comprend au moins les étapes suivantes :
a) la préparation de fragments d'ADN double-brin possédant des extrémités cohésives par digestion d'un échantillon d'acides nucléiques à analyser à l'aide d'au moins une première enzyme de restriction qui coupe l'ADN de façon décalée et qui possède un site de reconnaissance de l'ADN confondu avec le site de coupure de l'ADN, et simultanément à l'étape a),
b) la ligation des extrémités cohésives desdits fragments d'ADN à un adaptateur oligonucléotidique double-brin (adaptateur AA') comprenant successivement : une extrémité complémentaire de la séquence cohésive desdits fragments d'ADN (zone 1), immédiatement suivie par au moins une paire de bases différente de celle qui dans ledit site de coupure de la première enzyme de restriction précède directement la séquence complémentaire correspondant à la zone 1, et le site de reconnaissance d'une seconde enzyme de restriction dont le site de coupure est situé en aval dudit site de reconnaissance (zone 2),
c) l'amplification des fragments liés audit adaptateur, à l'aide d'un couple d'amorces appropriées, et
d) la coupure desdits fragments d'ADN à proximité d'une de leurs extrémités, à l'aide de la seconde enzyme de restriction, de façon à générer des fragments courts.

2. Procédé de préparation de fragments d'ADN selon la revendication 1, **caractérisé en ce que** ledit adaptateur comprend, en amont du site de reconnaissance (zone 2), une zone 3 d'au moins 6 pb.

3. Procédé de préparation de fragments d'ADN selon la revendication 1 ou la revendication 2, **caractérisé en ce que** ledit adaptateur comprend un résidu de phosphate lié de façon covalent à l'extrémité 5' du brin A'.

4. Procédé de préparation de fragments d'ADN selon l'une quelconque des revendications 1 à 3, **caractérisé ce que** l'une des amorces est liée à son extrémité 5' à un marqueur approprié.

5. Procédé de préparation de fragments d'ADN selon l'une quelconque des revendications 1 à 4, **caractérisé en ce que** lesdites amorces contiennent à leur extrémité 3', plusieurs bases spécifiques de séquence(s) informative(s) à détecter.

6. Procédé de préparation de fragments d'ADN selon l'une quelconque des revendications 1 à 5, **caractérisé en ce que** l'un des brins du produit amplifié à l'étape c) est protégé à son extrémité 5' par un marqueur approprié.

7. Procédé de préparation de fragments d'ADN selon l'une quelconque des revendications 1 à 6, **caractérisé en ce qu'**il comprend une étape additionnelle e) d'obtention, par tout moyen approprié, de fragments simple-brin à partir des fragments courts obtenus à l'étape d).

8. Procédé de préparation de fragments d'ADN selon l'une quelconque des revendications 1 à 7, **caractérisé en ce qu'**il comprend une étape additionnelle e') de purification des fragments courts obtenus à l'étape d) ou une étape f) de purification des fragments simple-brin obtenus à l'étape e).

9. Utilisation d'un adaptateur tel que défini à l'une quelconque des revendications 1 à 3, pour la préparation de courts fragments d'ADN simple-brin.

10. Amorce, **caractérisée en ce que** sa séquence est sélectionnée dans le groupe constitué par : la séquence de l'oligonucléotide A de l'adaptateur tel

11. Adaptateur, **caractérisé en ce qu'**il est formé d'un oligonucléotide double-brin (AA') d'au moins 10 pb comprenant successivement, de l'une à l'autre de ses extrémités :
- une zone 3 d'au moins 6 pb,
- une zone 2 comprenant le site de reconnaissance d'une enzyme de restriction dont le site de coupure est situé en aval du site de reconnaissance, et
- une zone 1 complémentaire des extrémités cohésives des fragments d'ADN double-brin de l'étape a) de la revendication 1, immédiatement précédée d'au moins une paire de bases différente de celle qui dans le site de coupure de la première enzyme de restriction précède directement la séquence complémentaire de la zone 1, ladite zone 1 comprenant un résidu de phosphate lié de façon covalente à l'extrémité 5' du brin A'.

12. Utilisation d'un couple d'amorces tel que défini à la revendication 10, pour la préparation de courts fragments d'ADN simple-brin. que défini à la revendication 1, et la séquence de ce dernier, additionnée en 3' des bases correspondants à la séquence cohésive desdits fragments d'ADN fragments d'ADN double-brin de l'étape a) de la revendication 1.

13. Kit pour la mise en oeuvre du procédé selon l'une quelconque des revendications 1 à 8, **caractérisé en ce qu'**il comprend au moins un adaptateur selon la revendication 11 ou une amorce selon la revendication 10.

## Claims

1. A method of preparing DNA fragments, **characterized in that** it comprises at least the following steps:
a) preparing double-stranded DNA fragments with cohesive ends, by digestion of a nucleic acid sample to be analyzed with at least one first restriction enzyme which makes staggered cuts in the DNA and whose DNA recognition site coincides with the DNA cleavage site, and simultaneously to step a),
b) ligating the cohesive ends of said DNA fragments to a double-stranded oligonucleotide adaptor (adaptor AA') comprising successively: one end complementary to the cohesive sequence of said DNA fragments (zone 1), followed immediately by at least one base pair that is different from that which in the cleavage site of said first restriction enzyme, precedes directly the complementary sequence corresponding to zone 1, and the recognition site for a second restriction enzyme, the cleavage site of which is located downstream of said recognition site (zone 2),
c) amplifying the fragments linked to said adaptor, using a pair of suitable primers, and
d) cleaving said DNA fragments close to one of their ends, using said second restriction enzyme, so as to generate short fragments.

2. The method of preparing DNA fragments as claimed in claim 1, **characterized in that** said adaptor comprises, upstream of the recognition site (zone 2), a zone 3 of at least 6 bp.

3. The method of preparing DNA fragments as claimed in claim 1 or claim 2, **characterized in that** said adaptor comprises a phosphate residue covalently linked to the 5' end of the strand A'.

4. The method of preparing DNA fragments as claimed in any one of claims 1 to 3, **characterized in that** one of the primers is linked at its 5' end to a suitable label.

5. The method of preparing DNA fragments as claimed in any one of claims 1 to 4, **characterized in that** said primers contain, at their 3' end, several bases specific for informative sequence(s) to be detected.

6. The method of preparing DNA fragments as claimed in any one of claims 1 to 5, **characterized in that** one of the strands of the product amplified in step c) is protected at its 5' end with a suitable label.

7. The method of preparing DNA fragments as claimed in any one of claims 1 to 6, **characterized in that** it comprises an additional step e) consisting in obtaining, by any suitable means, single-stranded fragments from the short fragments obtained in step d).

8. The method of preparing DNA fragments as claimed in any one of claims 1 to 7, **characterized in that** it comprises an additional step e'), consisting in purifying the short fragments obtained in step d), or a step f) consisting in purifying the single-stranded fragments obtained in step e).

9. The use of an adaptor as defined in any one of claims 1 to 3 for preparing short single-stranded DNA fragments.

10. A primer, **characterized in that** its sequence is selected from the group consisting of: the sequence of the oligonucleotide A of the adaptor as defined in claim 1, and the sequence of the latter, to which are added, in the 3' position, bases corresponding to the cohesive ends of said double-stranded DNA fragments from step a) of claim 1.

11. An adaptor, **characterized in that** it is formed from a double-stranded oligonucleotide (AA') of at least 10 bp comprising successively, from one to the other of its ends :
- a zone 3 of at least 6 bp,
- a zone 2 comprising the recognition site for a restriction enzyme, the cleavage site of which is located downstream of the recognition site, and
- a zone 1 complementary to the cohesive ends of said DNA fragments from step (a) of claim 1, preceded immediately by at least one base pair that is different from that which in the cleavage site of said first restriction enzyme, precedes directly the complementary sequence of zone 1, said zone 1 comprising a phosphate residue covalently linked to the 5' end of the strand A'.

12. The use of a pair of primers as defined in claim 10, for preparing short single-stranded DNA fragments.

13. A kit for carrying out the method as claimed in any one of claims 1 to 8, **characterized in that** it comprises at least one adaptor as claimed in claim 11 or a primer as claimed in claim 10.

## Patentansprüche

1. Verfahren zur Herstellung von DNA-Fragmenten, **dadurch gekennzeichnet, dass** es wenigstens die folgenden Stufen umfasst:
a) die Herstellung von Fragmenten doppelsträngiger DNA, kohäsive Enden besitzend, durch Verdau einer zu analysierenden Probe von Nucleinsäuren mit Hilfe wenigstens eines ersten Restriktionsenzyms, das die DNA in versetzter Weise schneidet und das eine Erkennungsstelle für die DNA besitzt, die mit der Schnittstelle der DNA verschmolzen ist, und gleichzeitig mit Stufe a)
b) die Ligation der kohäsiven Enden der DNA-Fragmente mit einem doppelsträngigen Oligonucleotid-Adapter (Adapter AA'), der aufeinanderfolgend umfasst: ein komplementäres Ende der kohäsiven Sequenz der DNA-Fragmente (Zone 1), unmittelbar gefolgt von wenigstens einem Basenpaar, das von dem verschieden ist, das in der Schnittstelle des ersten Restriktionsenzyms direkt der komplementären Sequenz, die Zone 1 entspricht, vorausgeht, und die Erkennungsstelle eines zweiten Restriktionsenzyms, dessen Schnittstelle stromabwärts der Erkennungsstelle (Zone 2) liegt,
c) die Amplifikation der an den Adapter gebundenen Fragmente mit Hilfe eines Paars geeigneter Primer und
d) das Schneiden der DNA-Fragmente in der Nähe eines ihrer Enden mit Hilfe des zweiten Restriktionsenzyms derart, dass kurze Fragmente erzeugt werden.

2. Verfahren zur Herstellung von DNA-Fragmenten gemäß Anspruch 1, **dadurch gekennzeichnet, dass** der Adapter stromaufwärts der Erkennungsstelle (Zone 2) eine Zone 3 aus wenigstens 6 bp umfasst.

3. Verfahren zur Herstellung von DNA-Fragmenten gemäß Anspruch 1 oder Anspruch 2, **dadurch gekennzeichnet, dass** der Adapter einen Phosphatrest kovalent an das 5'-Ende des Strangs A' gebunden umfasst.

4. Verfahren zur Herstellung von DNA-Fragmenten gemäß einem der vorangehenden Ansprüche, **dadurch gekennzeichnet, dass** einer der Primer an seinem 5'-Ende an einen geeigneten Marker gebunden ist.

5. Verfahren zur Herstellung von DNA-Fragmenten gemäß einem der Ansprüche 1 bis 4, **dadurch gekennzeichnet, dass** die Primer an ihrem 3'-Ende mehrere spezifische Basen einer Sequenz (von Sequenzen), die zum Detektieren informativ ist (sind), enthalten.

6. Verfahren zur Herstellung von DNA-Fragmenten gemäß einem der Ansprüche 1 bis 5, **dadurch gekennzeichnet, dass** einer der Stränge des in Stufe c) amplifizierten Produkts an seinem 5'-Ende durch einen geeigneten Marker geschützt wird.

7. Verfahren zur Herstellung von DNA-Fragmenten gemäß einem der Ansprüche 1 bis 6, **dadurch gekennzeichnet, dass** es eine zusätzliche Stufe e) zum Erhalt, durch jedes geeignete Mittel, von Einzelstrangfragmenten aus den in Stufe d) erhaltenen kurzen Fragmenten umfasst.

8. Verfahren zur Herstellung von DNA-Fragmenten gemäß einem der Ansprüche 1 bis 7, **dadurch gekennzeichnet, dass** es eine zusätzliche Stufe e') der Reinigung der kurzen Fragmente, die in Stufe d) erhalten wurden, oder eine Stufe f) der Reinigung der in Stufe e) erhaltenen Einzelstrangfragmente umfasst.

9. Verwendung eines Adapters, wie er in einem der Ansprüche 1 bis 3 definiert ist, für die Herstellung von kurzen Fragmenten einzelsträngiger DNA.

10. Primer, **dadurch gekennzeichnet, dass** seine Sequenz ausgewählt ist aus der Gruppe, bestehend aus: der Sequenz des Oligonucleotids A des Adapters, wie in Anspruch 1 definiert, und der Sequenz dieses Letztgenannten mit einer Addition an 3' der Basen, die der kohäsiven Sequenz der DNA-Fragmente entsprechen, von Fragmenten doppelsträngiger DNA der Stufe a) von Anspruch 1.

11. Adapter, **dadurch gekennzeichnet, dass** er aus einem doppelsträngigen Oligonucleotid (AA') aus wenigstens 10 bp gebildet ist, umfassend sukzessive von dem einen zu dem anderen seiner Enden:
- eine Zone 3 aus wenigstens 6 bp,
- eine Zone 2, umfassend die Erkennungsstelle eines Restriktionsenzyms, dessen Schnittstelle sich stromabwärts der Erkennungsstelle befindet, und
- eine komplementäre Zone 1 der kohäsiven Enden der Fragmente doppelsträngiger DNA der Stufe a) nach Anspruch 1, der unmittelbar wenigstens ein anderes Basenpaar vorausgeht als das, das in der Schnittstelle des ersten Restriktionsenzyms unmittelbar der komplementären Sequenz der Zone 1 vorausgeht, wobei die Zone 1 einen Phosphatrest in kovalenter Art an das 5'-Ende des Strangs A' gebunden umfasst.

12. Verwendung eines Primerpaars, wie es in Anspruch 10 definiert ist, zur Herstellung von kurzen Fragmenten einzelsträngiger DNA.

13. Kit zur Durchführung des Verfahrens gemäß einem der Ansprüche 1 bis 8, **dadurch gekennzeichnet, dass** er wenigstens einen Adapter gemäß Anspruch 11 oder einen Primer gemäß Anspruch 10 umfasst.


### SEQUENCE LISTING

<110> commissariat à l'Energie Atomique BRACHET, Anne-Gaelle RIZO, Philippe
<120> Procédé de préparation de fragments d'ADN et ses applications
<130> s263PCT90
<150> FR 0303294
   <151> 2003-03-18
<160> 12
<170> PatentIn version 3.1
<210> 1
   <211> 17
   <212> DNA
   <213> artificial sequence
<220>
   <223> adaptateur A
<400> 1
   ggaagcctag ctggagc 17
<210> 2
   <211> 21
   <212> DNA
   <213> artificial sequence
<220>
   <223> adaptateur A'
<400> 2
   aattgctcca gctaggcttc c 21
<210> 3
   <211> 21
   <212> DNA
   <213> artificial sequence
<220>
   <223> amorce B
<400> 3
   ggaagcctag ctggagcaat t 21
<210> 4
   <211> 128
   <212> DNA
   <213> artificial sequence
<220>
   <223> fragment long fl1
<400> 4
   cgatgagtgc tgaccgagct gtatttggga cctggggtct tttccgaacg atgcacggag 60
   aaactgcccc ttcgtgttga cttcattcac agggtggagt tcggagaggt gtccgcatcg 120
   cagtctac 128
<210> 5
   <211> 157
   <212> DNA
   <213> artificial sequence
<220>
   <223> fragment long fl2
<400> 5
   cgatgagtgc tgaccgagct gtatttggaa cctggggttt tttccgaacg atgcacggaa 60
   aaactgcccc ttcgtgttga ctgcattcac aggctggagt tcggagaggt gtccgggcat 120
   cgggttctta tcaagagggg accgcatcgc agtctac 157
<210> 6 <211> 49

   <212> DNA
   <213> artificial sequence
<220>
   <223> fragment long fl4
<400> 6
   cgatgagtgc tgaccctctc tccggcgaac agcccgcatc gcagtctac 49
<210> 7
   <211> 89
   <212> DNA
   <213> artificial sequence
<220>
   <223> fragment long f15
<400> 7
   cgatgagtgc tgaccgactc tttgcaaccc tgtgaatccc agcacgccag gcctccctgt 60
   ccatcaccaa ctcccgcatc gcagtctac 89
<210> 8
   <211> 17
   <212> DNA
   <213> artificial sequence
<220>
   <223> amorce
<400> 8
   cgatgagtgc tgaccga 17
<210> 9
   <211> 15
   <212> DNA
   <213> artificial sequence
<220>
   <223> amorce
<400> 9
   gtagactgcg atgcg 15
<210> 10
   <211> 13
   <212> DNA
   <213> artificial sequence
<220>
   <223> amorce
<400> 10
   cgatgagtgc tga 13
<210> 11
   <211> 17
   <212> DNA
   <213> artificial sequence
<220>
   <223> amorce
<400> 11
   cgatgactgg agaccga 17
<210> 12
   <211> 17
   <212> DNA
   <213> artificial sequence
<220>
   <223> amorce
<400> 12
   cgatgagttc cgaccga 17